# EUROPEAN PATENT APPLICATION

(11) **EP 4 702 953 A1**
(43) Date of publication of application: **04.03.2026**
(21) Application number: 24197403.9
(22) Date of filing: 29.08.2024
(51) Int. Cl.: A61F 11/08

(54) **AN EAR PLUG**

(71) Applicant: Loop, 2600 Berchem (BE)
(72) Inventor: Bijl, Hendrik Jouke, 1393 NG Nigtevecht (NL); Moens, Siemen, 2880 Bornem (BE); De Rydt, Tim, 2970 's Gravenwezel (BE); Veldhuis, Evert, 1095 HV Amsterdam (NL); Grainger, William, 2600 Berchem (BE)
(74) Representative: Hoyng Rokh Monegier B.V.

(57) **Abstract**

An ear plug (100) comprises an outer ear part comprising a stem (110). The ear plug (100) comprises an ear tip (200) coupled to the stem (110) of the outer ear part an ear tip (200) coupled to the stem (110). The outer ear part comprises:
- a substrate (102) defining the shape and providing the stem (110) of the ear plug (100), the substrate (102) comprising a first and a second portion (111, 112); and
- a covering layer (120) covering said first portion (111) of the substrate (102) and providing the outer surface of said first portion (111), the outer end (113) of the stem (110) being provided by the second portion (112) of the substrate (102) and being adapted to be inserted in the ear tip (200), said second portion (112) being free of said covering layer (120).

The substrate (102) is provided from a first polymer, the covering layer (120) being provided from a second polymer being a silicone polymer, the hardness of the first polymer being larger than the hardness of the second polymer.

## Description

### Field of the Invention

The present invention generally relates to ear plugs, ear tips of ear plugs and methods to provide ear plugs.

### Background of the Invention

Ear plugs nowadays are used very extensively and possibly in almost any circumstances. Ear plugs are used at home during sleeping, working or studying, or at work in the offices where e.g. the office is organized as landscape offices, and this in order to give the user a sufficient lever of silence allowing to concentrate to his or her work. Ear plugs are used in production halls to prevent hearing damage due to loud noise from machines or processes being ongoing, or in the streets to avoid the traffic noise. Ear plugs are used during concerts to avoid hearing damage due to too high sound volumes being used. In fact, ear plugs are used as good as anywhere.

This is why the requirements for good ear plugs have increased over the last decades. Ear plugs need to provide physical comfort to the user when using the ear plug. The ear plug needs to provide a good and firm connection between body (more particularly the ear channel) and the ear tip, to avoid a too low pull out force. Ear plugs needs to be easy to assemble and disassemble to allow cleaning and maintenance. Ear plugs needs to be easy to insert in the ear channel as well.

Hence a very high quality demand with often possibly conflicting expectations and requirements are met.

### Summary of the Invention

According to a first aspect of the invention, an ear plug is provided. The ear plug comprises an outer ear part comprising a stem. The ear plug comprises an ear tip coupled to the stem of the outer ear part. The outer ear part comprises:
- a substrate defining the shape and providing the stem of the ear plug, the substrate comprising a first and a second portion; and
- a covering layer covering said first portion of the substrate and providing the outer surface of said first portion, the outer end of the stem being provided by the second portion of the substrate and being adapted to be inserted in the ear tip, said second portion being free of said covering layer.

The substrate is provided from a first polymer, the covering layer being provided from a second polymer being a silicone polymer, the hardness of the first polymer being larger than the hardness of the second polymer.

The outer ear part may consist of the first and second portion. Possibly the substrate has the shape of the outer portion of the ear plug. Hence the outer ear portion may be completely covered with the covering layer, except the second portion which may be inserted completely in the ear tip. The substrate may hence function - so to say - as the backbone of the ear plug.

By providing a substrate from a harder polymer, the covering being provided by are relatively softer polymer, the harder substrate may provide the strength and stiffness of the ear plug, allowing the plug to be positioned and inserted in the ear channel correctly, whereas the covering layer may cause the ear plug to be easier to use, softer, more pleasant and more comfortable to wear.

The second polymer is a silicone polymer, although also other soft polymers may be used. Silicone polymers are better tolerated by the end users having a more comfortable skin contact.

First polymer may be one or more polymers chosen from the group of thermoplastic elastomers (TPE), e.g. thermoplastic polyurethane (TPU), rubber like polymers like natural rubber, nitrile rubber, Ethylene-Propylene-Diene-Monomer (EPDM) rubbers, Acrylonitrile-butadiene-styrene (ABS), Polycarbonate/acrylonitrile butadiene styrene (PC-ABS), silicone rubbers (also referred to as silicone), Polyesters (PES), polyamide (PA, like PA6, PA6.6, PA6.10, and alike), Acrylic or Polymethyl Methacrylate (PMMA), Polycarbonate (PC), Polyethylene (PE), Polypropylene (PP), Polyethylene Terephthalate (PETE or PET), Polyvinyl Chloride (PVC), Polyoxymethylene (POM), and similar and combinations of two or more of these polymers.

Silicone polymer is also referred to as polysiloxane, and is the preferred polymer. The polymer may comprise additives such as organic or inorganic fillers. In order to modify the hardness of the silicone polymers, more or less silicone oils or gum, more or less or alternative fillers or more or less couplers or cross linking agents may be added, higher or lower molecular weight for the silicone polymers may be used, and/or the curing time of the polymer may be modified.

The stem of the outer ear part of the ear plug is the section of the outer ear part, which extends inwards the ear channel when the ear plug is worn. To the outer end of the stem, the eat tip is connected. Usually the ear tip is plugged on the outer end of the stem. The ear tip may comprise a void space, preferably substantially tubular shaped, to receive the outer end of the stem and allow the outer end of the stem to snuggly fit in this void space. The wall of the void space and the stem may fit together so the ear tip is kept on the stem due to a sufficiently high friction force between wall of the void space and the stem.

The stem may be provided with a uniform or gradually changing radial cross section. The provision of a uniform radial cross section is preferred.

The radial cross section or section of the stem may be circular or non-circular. A non-circular radial cross section is preferred. The non-circular radial cross section may e.g. be oval (egg-shaped), stadium or racetrack shaped, or elliptic. Most preferably the cross section itself has at maximum one axis of symmetry and preferably has no point of symmetry. Such non-circular radial cross sections may provide a limited number of, and possibly only one orientation and/or position in which the stem can be inserted in a void space in the ear tip, which has the same radial cross section of the stem. Preferably significant differences in widths, measurable in the same cross section are provided, to obtain the best results. As an example, in case of a stadium shape, the length of the two axes of symmetry are preferably substantially different. The non-circular radial cross sections may comprise one or more recesses and/or one or more protrusions, which may serve as steering elements and may cooperate with one or more protrusions respectively one or more recesses in the surface of the ear tip in which the stem is to be inserted. By means of these recesses and/or protrusions, the steering elements move, steer and assist or guide the two components correctly towards one another. Preferably significantly large protrusions or recesses, measurable in the same cross section are provided, to obtain the best results. The steering elements may center the stem and the ear tip relative to one another.

Hence according to some embodiments, the stem may have a non-circular radial cross section. According to some embodiments, the stem may have an oval, stadium or racetrack shaped or elliptic radial cross section.

The hardness of the first polymer is larger than the hardness of the second polymer. Hereinafter, unless explicitly mentioned differently, hardnesses are expressed in Shore A, measured using a durometer.

Depending on the polymers used, some polymers' hardness can only be measured using a durometer expressing a Shore A value, while other polymers' hardness can only be expressed in Shore D values. And for some polymers, the hardness can be expressed by two values, being a Shore A value and a Shore D value. Polymers having a hardness which can only be expressed by a Shore A value are less hard than polymers having a hardness which can only be expressed by a Shore D value. In case the first and or the second polymer is a polymer whose hardness can be expressed by both a Shore A value and a Shore D value, the hardness comparison must be made using the Shore value which can be measured for both polymers, hence either being the common Shore A or the common Shore D. In case the hardness of both polymers, i.e. the first and the second polymer, is measurable using a Shore A and Shore D value, the Shore A values are to be used for comparison.

Preferably the first and second polymer have a hardness which can be expressed by a Shore A value. According to some embodiments, the Shore A hardness difference between the first polymer and the second polymer may be in the range of 30 to 80. More preferred, the Shore A hardness difference between the first polymer and the second polymer may be in the range of 40 to 75, such as in the range of 50 to 75, e.g. 65, 66, 67, 68, 69, 70, 71, 72, 73, 74 or 75.

According to some embodiments, the Shore A hardness of the first polymer may range from of 50 to 100. More preferred, the hardness of the first polymer may be in the range of 65 to 95, such as in the range of 70 to 90.

According to some embodiments, the Shore A hardness of the second polymer may range from of 10 to 50. More preferred, the Shore A hardness of the second polymer may be in the range of 15 to 45, such as in the range of 20 to 40.

The second polymer may be one or more polymers chosen from the group of thermoplastic elastomers (TPE), e.g. thermoplastic polyurethane (TPU), rubber like polymers like natural rubber, nitrile rubber, Ethylene-Propylene-Diene-Monomer (EPDM) rubbers, Acrylonitrile-butadiene-styrene (ABS), Polycarbonate/acrylonitrile butadiene styrene (PC-ABS), silicone rubbers (also referred to as silicone), Polyesters (PES), polyamide (PA, like PA6, PA6.6, PA6.10, and alike), Acrylic or Polymethyl Methacrylate (PMMA), Polycarbonate (PC), Polyethylene (PE), Polypropylene (PP), Polyethylene Terephthalate (PETE or PET), Polyvinyl Chloride (PVC), Polyoxymethylene (POM), and similar and combinations of two or more of these polymers.

The thickness of the second polymer layer, i.e. the covering layer may have a uniform or a non-uniform thickness over the first portion of the substrate.

According to some embodiments, the thickness of the second polymer layer, i.e. the covering layer, may have a non-uniform thickness over the first portion of the substrate.

According to some embodiments, the thickness of the second polymer layer being the covering layer may be in the range of 1mm to 10mm.

More preferred, the thickness of the second polymer layer, i.e. the covering layer, may be in the range of 2mm to 6.5mm, such as in the range of 3.3mm to 3.9mm.

According to some embodiments, the average thickness of the second polymer layer, i.e. the covering layer, may be in the range of 1mm to 10mm. More preferred, the average thickness of the second polymer layer, i.e. the covering layer may be in the range of 2mm to 6.5mm, such as in the range of 3.3mm to 3.6mm.

The ear plugs according to the first aspect of the invention provide a high physical comfort to the user, seen the soft touch and feel of the second polymer of the covering layer, while the relatively harder, and hence stiffer first polymer allows the provision of a sufficiently stiff ear plug to insert and guide the ear tip into the ear channel. The second, non-covered portion of the stem allows an easy insertion of the stem to the ear tip, hence a gentle assembling and disassembling to clean the ear tip. The preferred non-circular cross section allows a guiding of the stem during plugging the ear tip to the stem. The tuning of the hardness of the polymer of the stem and the polymer of the ear tip may provide a proper friction force ensuring the ear tip to stay firm on the stem, while the polymer used for the ear tip itself provides a sufficient connection between ear channel and ear tip, thereby tuning the pull-out force of the ear tip and hance the ear plug. The possible reduced radial cross section of the stem may allow sufficiently stiffness to insert the ear plug in the ear channel, while being slim enough to both provide space for an outer soft polymer layer providing a good and comfortable feeling, and allow flexing of the stem when the user is e.g. a side sleeping person, which needs the ear plug to bend and flex to allow good sleeping comfort while wearing the ear plug.

According to a second aspect of the invention, an ear tip is provided, the ear tip comprises:
∘ an outer shell for contacting an ear channel when inserted in an ear;
∘ a tubular hollow element within the outer shell, said element extending in a direction outwards the ear channel when inserted in an ear channel and adapted for receiving the stem of an outer ear part of an ear plug,
∘ a volume between the outer shell and the tubular hollow element being at least partially filled with foamed polymer.

The outer shell preferably has a cup shape and may preferably be provided from silicone polymer. Possible polymer may be one or more polymers chosen from the group of thermoplastic elastomers (TPE), e.g. thermoplastic polyurethane (TPU), rubber like polymers like natural rubber, nitrile rubber, Ethylene-Propylene-Diene-Monomer (EPDM) rubbers, Acrylonitrile-butadiene-styrene (ABS), Polycarbonate/acrylonitrile butadiene styrene (PC-ABS), silicone rubbers (also referred to as silicone), Polyesters (PES), polyamide (PA, like PA6, PA6.6, PA6.10, and alike), Acrylic or Polymethyl Methacrylate (PMMA), Polycarbonate (PC), Polyethylene (PE), Polypropylene (PP), Polyethylene Terephthalate (PETE or PET), Polyvinyl Chloride (PVC), Polyoxymethylene (POM), and similar and combinations of two or more of these polymers.

The shell and/or the tubular hollow element preferably are provided from softer polymer as compared to the stem to which the ear tip is to be fixed upon. The polymer, preferably silicone polymer, of the shell and/or the tubular hollow element has a Shore A hardness in the range of 40 to 70, such as about 50. This higher hardness of the stem is to provide sufficient pressure to the stem when the stem is plugged in the tubular hollow element, thereby providing a significantly high pull out force to be exercised to remove the stem from the ear tip.

According to some embodiments, the shell may have a cup shape. Possibly, but not necessarily, the thickness of the shell may be uniform. However more preferably the thickness of the shell varies. The shell, having a cup-like shape, may have the thickest thickness at the bottom of the cup-shape, the thickness decreasing over the surface from the bottom to the upper rim of the cup-like shape. At the rim itself, the thickness may be increased again locally. As an example, at the bottom of the cup-like shape, the thickness of the shell may be e.g. in the range of 0.4 to 1.5mm, e.g. 0.7mm. The thickness may decrease over the surface from the bottom to the upper rim of the cup-like shape down to a thickness in the range of 0.6 to 0.1mm, e.g. down to 0.3mm. At the rim itself the thickness may be increased again locally to a range of 0.4 to 1.0mm, e.g. to 0.7mm.

According to some embodiments, the shell may have an average thickness in the range of 0.2mm to 1.5mm. More preferred, the average thickness of the shell may be in the range of 0.25mm to 1.0mm, such as in the range of 0.3mm to 0.7mm, e.g.in the range of 0.3mm to 0.5mm.

This ear tip may be part of the ear plug according to the first aspect of the invention.

The radial cross section or section of the inner wall of the tubular hollow element may be circular or non-circular. The radial cross section of the inner wall of the tubular hollow element of the ear tip may correspond with the radial cross section of the outer end of the stem provided by the second portion of the substrate. The radial cross section of the inner wall of the tubular hollow element of the ear tip and the radial cross section of the perimeter of the outer end of the stem provided by the second portion of the substrate may be substantially identical. As such the tubular hollow element of the ear tip may snuggly fit on the outer end of the stem provided by the second portion of the substrate, and may be coupled to it by a sufficiently high friction force between the two surfaces.

According to some embodiments, the radial cross section or section of the inner wall of the tubular hollow element may be non-circular.

This tubular hollow element provides the void space of the ear tip, and is adapted to receive the outer end of the stem and allow the outer end of the stem to snuggly fit in this void space. The wall of the void space and the stem may fit together so the ear tip is kept on the stem due to a sufficiently high friction force between wall of the void space and the stem.

A non-circular radial cross section is preferred. The non-circular radial cross section may e.g. be oval (egg-shaped), stadium or racetrack shaped, or elliptic. Most preferably the cross section itself has at maximum one axis of symmetry and preferably has no point of symmetry. Most preferably the cross section itself has at maximum one axis of symmetry and preferably has no point of symmetry. Such non-circular radial cross sections may provide a limited number of, and possibly only one orientation and/or position in which a stem can be inserted in the void space in the ear tip.

The non-circular radial cross sections may comprise one or more recesses and/or one or more protrusions, which may serve as steering elements and may cooperate with one or more protrusions respectively one or more recesses on the radial cross section of the stem which is to be inserted. By means of these recesses and/or protrusions, the steering elements move, steer and assist or guide the two components correctly towards one another. The steering elements may center the stem and the ear tip relative to one another.

Possibly, but not necessarily, the thickness of the tubular hollow element is uniform and in a range of 0.2mm to 1.5mm, e.g. 0.9mm. at the rim of the tubular hollow element, the thickness may locally be less, e.g. in the range of 0.1 to 0.7mm, e.g. 0.7mm

According to some embodiments, the tubular hollow element may have an average thickness in the range of 0.1mm to 7mm.

More preferred, the average thickness of the tubular hollow element may be in the range of 0.4mm to 3mm, such as in the range of 0.5mm to 1.2mm.

The tubular hollow element may be provided from one or more polymers chosen from the group of thermoplastic elastomers (TPE), e.g. thermoplastic polyurethane (TPU), rubber like polymers like natural rubber, nitrile rubber, Ethylene-Propylene-Diene-Monomer (EPDM) rubbers, Acrylonitrile-butadiene-styrene (ABS), Polycarbonate/acrylonitrile butadiene styrene (PC-ABS), silicone rubbers (also referred to as silicone), Polyesters (PES), polyamide (PA, like PA6, PA6.6, PA6.10, and alike), Acrylic or Polymethyl Methacrylate (PMMA), Polycarbonate (PC), Polyethylene (PE), Polypropylene (PP), Polyethylene Terephthalate (PETE or PET), Polyvinyl Chloride (PVC), Polyoxymethylene (POM), and similar and combinations of two or more of these polymers. Silicone polymers are preferred.

The shell and the tubular hollow element may form one integral piece, possibly being an injection molded piece of polymer material.

The volume between the outer shell and the tubular hollow element is at least partially, and preferably completely filled with foamed polymer.

According to some embodiments, the foamed polymer may be a polyurethane foam. More preferably the foamed volume is provided from open cell foam or closed cell foam or a combination of open and closes call foam.

. The foam used to fill the volume may have a retarded thickness recovery. Hence preferably a viscoelastic foam is used, like viscoelastic PU foam. The recovery time preferably is in the range of 2 to 10 seconds, e.g. in the range of 3 to 7 seconds like 4 seconds, 5 seconds or 6 seconds. The foamed volume may have a volume in the range of 300 mm³ to 900 mm³, such as in the range of 350 mm³ to 800 mm³, e.g. in the range of 370 mm³ to 790 mm.

The tubular hollow element may be centered in the outer shell. Each of the tubular hollow element and the shell has an outer rim oriented towards the side of the ear tip oriented and facing outwards the ear channel in which the ear tip is adapted to be positioned.

According to some embodiments, the tubular hollow element may have an outer rim, said outer rim of the tubular hollow element is positioned within the volume defined by the shell.

According to a third aspect of the invention, a method to make an ear plug is provided, the method comprises the steps of
∘ providing an ear tip comprising a void space, adapted to receive the outer end of a stem of an outer ear part of the ear plug;
∘ providing a stem of the outer ear part of the ear plug from a first polymer;
∘ use said stem as substrate in an overmolding operation, and cover by overmolding a first portion of the substrate with a second polymer having a lower hardness as compared to the first polymer, and keep a second portion of the substrate being free of being covered by said second polymer;
∘ inserting the second portion of the substrate in the void space of the ear tip to provide the ear plug.

According to some embodiments, the step of providing a stem may comprise molding the stem.

According to some embodiments, molding the stem comprises:
∘ injection of a first polymer in a mold, this mold comprises at least a first and a second mold part,
∘ removing the first mold part, while holding the molded substrate in the second mold part;
∘ inserting the first portion of the substrate in a third mold and injection molding the second polymer in this further mold, thereby overmolding a first portion of the substrate with a second polymer, and providing ; and providing a second portion of the substrate being free of said second polymer;
∘ removing the so-obtained the outer ear part from the first and third mold part.

According to some embodiments, the method further may comprise the step of providing an ear tip to the second portion of the substrate.

According to some embodiments, the ear plug is an ear plug according to the first aspect of the invention.

According to some embodiments, the ear tip of the ear plug is an ear tip according to the second aspect of the invention.

Hence the method according to the third aspect of the invention may provide an ear plug according to the first aspect of the invention, optionally this ear plug according to the first aspect of the invention may comprise an ear tip according to the second aspect of the invention.

It is understood that features from one of the aspects of the invention may be combined with one or more, even all features of one or more of the other aspects of the invention.

In general, when for a feature, reference is made to a range, the range is to be understood inclusive, unless explicitly mentioned differently.

### Brief Description of the Drawings

Fig. 1 illustrates schematically a side view of the outer ear part of an ear plug according to the invention.
Fig. 2 is a schematically view of the outer ear part of the ear plug of figure 1, but seen from the backside of the ear plug.
Fig. 3 is a schematically view of a cross section of the outer ear part of the ear plug of figures 1 and 2.
Fig. 4 is a schematically see-through view of a cross section of the outer ear part of the ear plug of figures 1 and 2.
Fig. 5a and 5b are schematically views of a radial cross section of the stem of the ear plug of figure 1 and 2 according to the plane BB'.
Fig. 6 is a schematically view of the outer ear part of the ear plug of the previous figures, on which an ear tip according to the second aspect of the invention is slid.
Fig. 7 is a schematically view of the ear tip according to the second aspect of the invention as used in figure 6.
Fig 8a is a more detailed view of a cross section of the ear tip as used in figure 6 and 7, whereas Fig 8b is a more detailed view of a cross section of the ear tip of figure 8a and the outer ear part of the ear plug of the previous figures. In the different figures, the same reference sign refers to the same or a similar feature.

### Detailed Description of Embodiment(s)

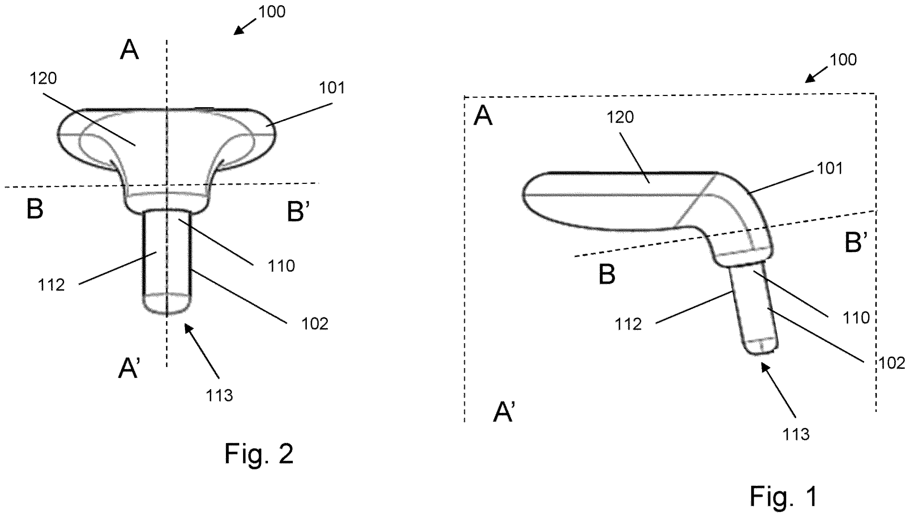

An ear plug 100 according to the present invention is shown in figures 1 to 6. Figure 1 is a side view of the outer ear part 101 of the ear plug 100. Also figure 2 is a view of the outer ear part 101 of the ear plug 100, but this time seen from the backside of the ear plug 100. The ear tip is not shown in figures 1 and 2.

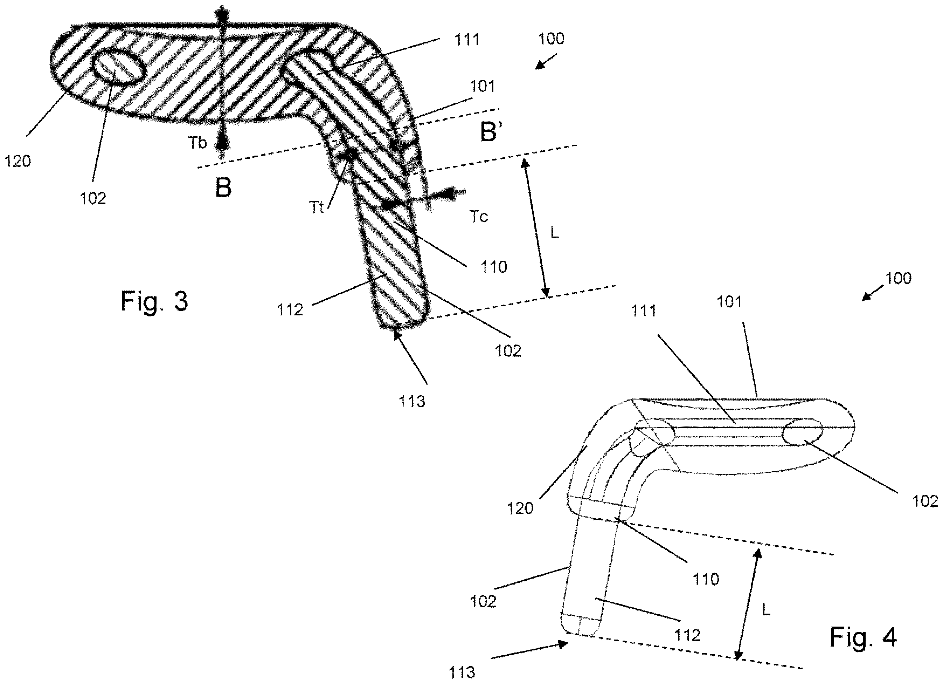

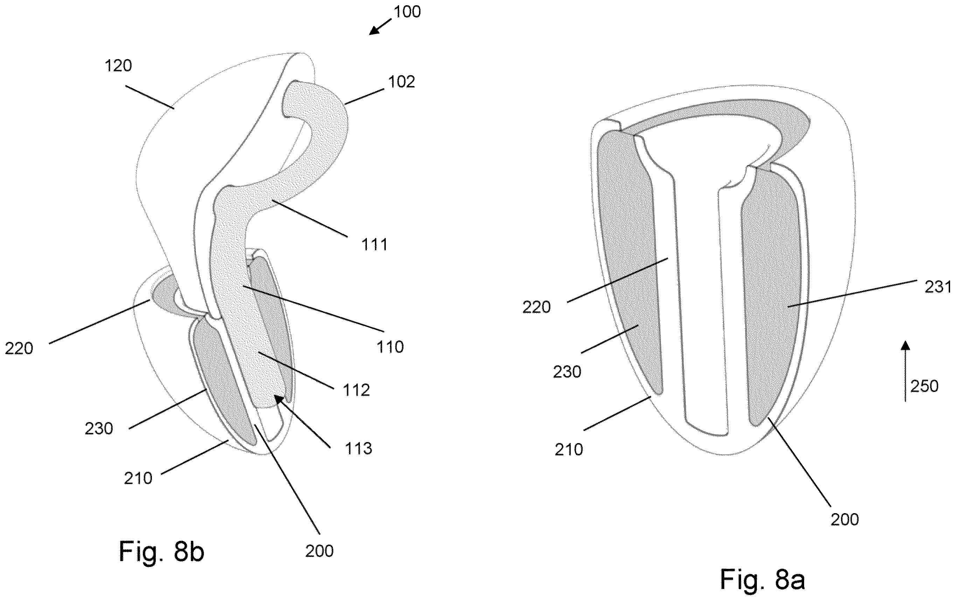

The outer ear part 101 comprises a substrate 102, which is best visible in figure 3, which figure is a cross section according to the plane AA', and in figure 8, where only part of the substrate 102 is shown as covered by a second polymeric layer being a covering layer 120.

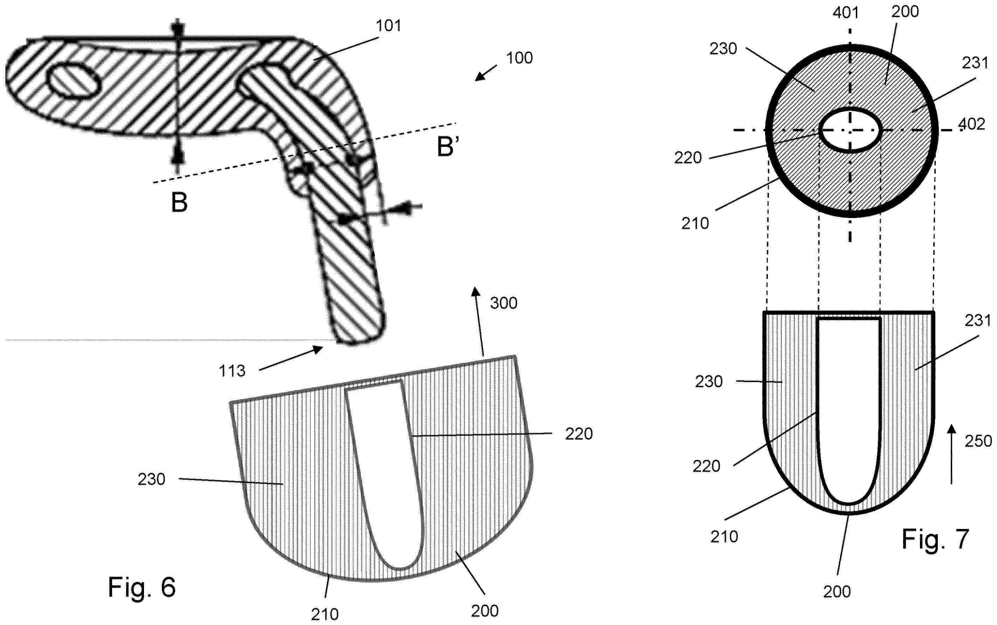

The outer ear part 101 comprising a stem 110. This stem 110, as shown in figures 6, 7 and 8, is coupled to an ear tip 200. The stem is provided as an injection molded silicone polymer with a hardness of about 90 Shore A.

The substrate 102 defining the shape and providing the stem 110 of the ear plug 100. The substrate 102 comprises a first portion 111 and a second portion 112. The first portion 111 is covered with a polymeric covering layer 120, being provided from a silicone polymer with hardness of about 20 Shore A. The polymeric covering layer 120 is provided over the first portion of the substrate by overmolding with this second polymer.

The outer end 113 of the stem 110 is provided by the second portion 112 of the substrate 102 being free of the covering layer. This second portion 112 of the substrate 102 is adapted to be inserted in the ear tip 200, as is shown in figure 6, where the second portion 112 of the substrate 102 slides in the ear tip 200 by moving the ear tip towards and over the outer end 113 in a direction 300.

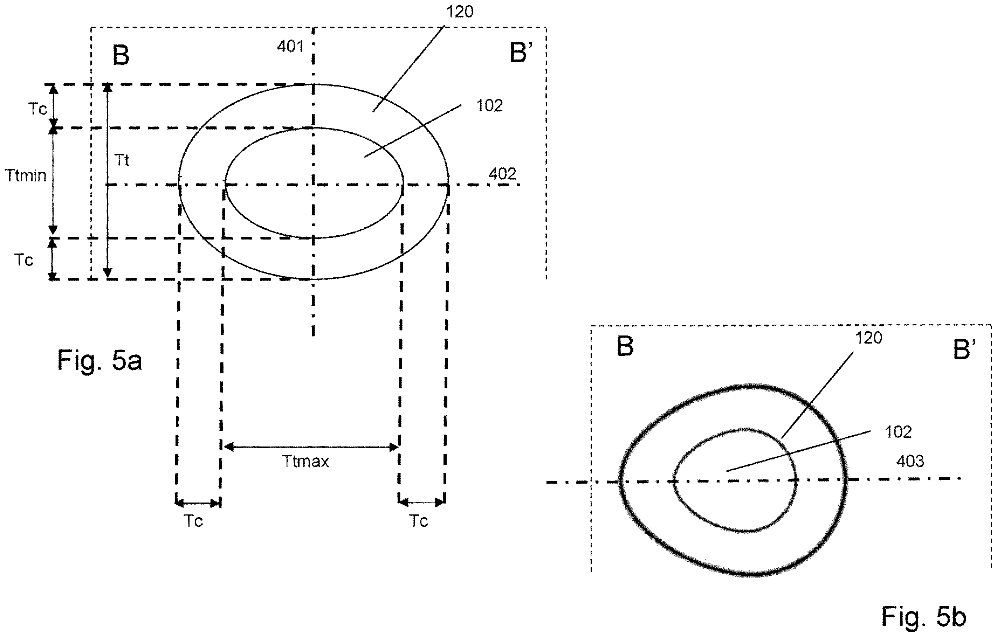

As best can be seen in figure 4 and 8, the substrate forms so-to-say the backbone of the ear plug 100. At the position of the stem 110, the radial cross section according to the plane BB', is non-circular. The radial cross section may e.g. be oval shaped with two axes of symmetry 401 and 402, as shown in figure 5a, or egg-shaped with one axis of symmetry 403 as shown in figure 5b. It is understood that the non-circular cross section may only be present at the second portion 112 of the substrate 102 where the substrate provides part of the stem 110, but, as shown in the figures, may already be applicable in a part of the first portion 111 of the substrate 102 where the substrate provides part of the stem 110.

The thickness of the covering layer may not be uniform all over the first portion 111 of the substrate 102. As a mere example, as shown in figure 3, in the part of the ear plug 100 which is to reside in the auricle during use, the total thickness of the ear plug Tb may be 3.6mm. The thickness of the substrate in this part may be 2.0 mm, hence the thickness of the covering layer is about 0.8mm. The portion of the stem where the stem is directed into the ear channel as shown in figure 3, is Tt. This is the smallest diameter of the total of the substrate and the covering layer. As better visible in figure 5a, the smallest diameter Ttmin of the substrate, seen the oval or egg-shaped cross section, is about 2.2mm, the largest Ttmax being 3.0mm, where a thickness Tc of the covering layer is about 0.8mm. The length L of the second portion of the stem is about 6.5mm.

The ear plug 100 is provided with an ear tip 200 according to the second aspect of the invention, as best shown in figure 8. The ear tip 200 comprises an outer shell 210 for contacting an ear channel when inserted in an ear, a tubular hollow element 220 within the outer shell 210, which element extends in a direction 250 outwards the ear channel when inserted in an ear channel. The volume 230 between the outer shell 210 and the tubular hollow element 220 is filled with foamed polymer 231.

The tubular hollow element 220 is adapted to receive the stem 110 of an outer ear part 101 of an ear plug 100, more particularly to receive the second portion 112 of the stem 110. Seen the non-circular radial cross section of the second portion 112 of the stem 110, and the corresponding non-circular radial cross section of the tubular hollow element 220, these two parts snuggly fit one into the other and in only a limited, in particularly in two orientations. The tubular hollow element 220 and the shell, which has a cup shape. The average thickness of the tubular hollow element 220 is about 0.9 mm. The thickness of the shell is about 0.7 at the bottom point of the cup-shape, about 0.5mm at the curved part of the shell, and about 0.3mm at the upright part. At the rim though, the thickness is again about 0.4 to 0.7mm. The polymer, preferably silicone polymer, of the shell and the tubular hollow element has a Shore A hardness of about 50. The foamed volume is provided from viscoelastic PU foam having a response or recovery time of 5 seconds.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. An ear plug comprising
• an outer ear part comprising a stem;
• an ear tip coupled to the stem;
said outer ear part comprises:
- a substrate defining the shape and providing the stem of the ear plug, the substrate comprising a first and a second portion; and
- a covering layer covering said first portion of the substrate and providing the outer surface of said first portion, the outer end of the stem being provided by the second portion of the substrate and being adapted to be inserted in the ear tip, said second portion being free of said covering layer,
wherein the substrate being provided from a first polymer, the covering layer being provided from a second polymer, the hardness of the first polymer being larger than the hardness of the second polymer.

2. An ear plug according to claim 1, wherein the second polymer is a silicone polymer.

3. An ear plug according to any one of the claims 1 to 2, wherein the first polymer is a silicone polymer.

4. An ear plug according to anyone of the preceding claims, wherein the stem has a non-circular radial cross section.

5. An ear plug according to claim 4, wherein the stem has an oval, stadium or racetrack shaped or elliptic radial cross section.

6. An ear plug according to anyone of the preceding claims, wherein the Shore A hardness difference between the first polymer and the second polymer is in the range of 30 to 80.

7. An ear plug according to anyone of the preceding claims, wherein the Shore A hardness of the first polymer ranges from of 50 to 100.

8. An ear plug according to anyone of the preceding claims, wherein the Shore A hardness of the second polymer ranges from of 10 to 40.

9. An ear plug according to any one of the receding claims, wherein the thickness of the covering layer has a non-uniform thickness over the first portion of the substrate.

10. An ear plug according to claim 9, wherein the thickness of the covering layer is in the range of 1mm to 10mm.

11. A method to make an ear plug, comprising the steps of
∘ providing an ear tip comprising a void space, adapted to receive the outer end of a stem of an outer ear part of the ear plug;
∘ providing a stem of the outer ear part of the ear plug from a first polymer;
∘ use said stem as substrate in an overmolding operation, and cover by overmolding a first portion of the substrate with a second polymer having a lower hardness as compared to the first polymer, and keep a second portion of the substrate being free of being covered by said second polymer;
∘ inserting the second portion of the substrate in the void space of the ear tip to provide the ear plug.

12. A method to make an ear plug according to claim 11, wherein the step of providing a stem comprises molding the stem.

13. A method to make an ear plug according to claim 12, wherein molding the stem comprises
∘ injection of a first polymer in a mold, this mold comprises at least a first and a second mold part,
∘ removing the first mold part, while holding the molded substrate in the second mold part;
∘ inserting the first portion of the substrate in a third mold and injection molding the second polymer in this further mold, thereby overmolding a first portion of the substrate with a second polymer, and providing ; and providing a second portion of the substrate being free of said second polymer;
∘ removing the so-obtained the outer ear part from the first and third mold part.

14. A method to make an ear plug according to any one of the claims 11 to 13, wherein method further comprises the step of providing an ear tip to the second portion of the substrate.

15. A method to make an ear plug according to any one of the claims 11 to 14, wherein the ear plug is an ear plug according to any one of the claims 1 to 10.
